# EUROPEAN PATENT SPECIFICATION

(11) **EP 3 875 960 B1**
(45) Date of publication and mention of the grant of the patent: **10.07.2024**
(21) Application number: 18938386.2
(22) Date of filing: 30.10.2018
(51) Int. Cl.: G01N 35/00, G01N 33/50, G01N 33/53, G01N 35/02

(54) **REACTION APPARATUS AND IMMUNOASSAY ANALYZER**
REAKTIONSVORRICHTUNG UND IMMUNOASSAY-ANALYSATOR
APPAREIL DE RÉACTION ET ANALYSEUR D'IMMUNOESSAI

(43) Date of publication of application: 08.09.2021
(73) Proprietor: Shenzhen Increcare Biotech Co. Ltd, Shenzhen, Guangdong 518055 (CN)
(72) Inventor: ZHANG, Zhen, Shenzhen, Guangdong 518055 (CN)
(74) Representative: Cabinet Laurent & Charras
(86) International application number: PCT/CN2018/112534
(87) International publication number: WO 2020/087238

(56) References cited:
- EP-A1- 3 561 515
- WO-A1-2018/126775
- CN-A- 106 645 765
- CN-A- 106 706 942
- CN-A- 107 942 085
- CN-U- 207 866 714

## Description

### TECHNICAL FIELD

The present application relates to a technical field of in vitro diagnostics, and in particular, to a reaction device and an immunoassay analyzer.

### BACKGROUND

A fully automatic immunoassay analyzer can quantitatively or qualitatively detect target analytes such as antibodies and antigens contained in a sample to be tested, for example, blood or the like. Generally, the sample to be tested and a reagent (or a reactant) are added into an empty reactor and then mixed and incubated, and then subjected to a bound-free (BF) operation (sometimes referred to simply as washing herein), and then a signal reagent is added into the reactor to measure an optical signal or electrical signal, so as to measure and analyze the target analytes contained in the sample to be tested. An example of such automatic analyzer can be found for example in patent WO 2018/126775 A1.

For a conventional immunoassay analyzer, due to a large volume of the reaction device, the entire immunoassay analyzer has the defect that the structure thereof is not compact.

### SUMMARY

The invention is defined by the appended claims.

According to various embodiments, a reaction device capable of improving the structural compactness is provided.

A reaction device includes:
a rotating disk configured to incubate, wash, and measure a reactant in the reactor;
a washing assembly located above the rotating disk, and capable of dispensing a washing solution into the reactor and drawing unbound ingredients in the reactor, and the washing assembly including a signal reagent dispensing portion configured to dispense a signal reagent into the reactor subjected to a BF operation;
a signal reagent mixing unit configured to mix the signal reagent in the reactor after adding the signal reagent;
a measuring device connected to the rotating disk, and configured to measure an optical signal of the reactant in the reactor after adding the signal reagent; and
a transferring assembly capable of transferring the reactor between the rotating disk and the signal reagent mixing unit.

An immunoassay analyzer includes any reaction device as described above.

Details of one or more embodiments of the present application are set forth in the attached drawings and description. Other features, purposes and advantages of the present application will become apparent from the description, and drawings.

### BRIEF DESCRIPTION OF THE DRAWINGS

In order to better describe and illustrate embodiments and/or examples disclosed herein, reference may be made to one or more drawings. Additional details or examples used to describe the drawings should not be considered as a limitation on the scope of any of the disclosed inventions, the currently described embodiments and/or examples, and the best modes of these things currently understood.
FIG. 1 is a plan structural schematic view of an immunoassay analyzer according to a first embodiment.
FIG. 2 is a perspective schematic view of a mixing device in FIG. 1.
FIG. 3 is a plan structural schematic view of an immunoassay analyzer according to a second embodiment.
FIG. 4 is a perspective schematic view of a mixing device in FIG. 3.
FIG. 5 is a flow chart block diagram of a serial type mixing method according to an embodiment.
FIG. 6 is a flow chart block diagram of a parallel type mixing method according to an embodiment.
FIG. 7 is a flow chart block diagram of a reagent pipetting method according to an embodiment.
FIG. 8 is a flow chart block diagram of a diluting method according to an embodiment.
FIG. 9 is a flow chart block diagram of an immune analysis method according to an embodiment.

### DETAILED DESCRIPTION OF THE EMBODIMENTS

In order to facilitate the understanding of the present application, the present application will be described more fully below with reference to the relevant drawings. Preferred embodiments of the present application are shown in the drawings. However, the present application can be implemented in many different forms and is not limited to the embodiments described herein. On the contrary, providing these embodiments is to make the disclosure of the present application more thorough and comprehensive.

It should be understood that when an element is referred to as being "fixed on" another element, it can be directly on another element or intervening elements may be present therebetween. When an element is referred to as being "connected" or "coupled" to another element, it can be directly connected or coupled to the other element or intervening elements may be present therebetween. Terms "inside", "outside", "left", "right" and similar expressions used herein are for illustrative purposes only, and do not mean that they are the only embodiments.

Incubation of a sample and a reagent (or a reactant) specifically refers to a process of antigen-antibody binding reaction or biotin avidin binding reaction of the reactant in a reactor in a constant temperature environment before starting to be washed (undergo a bound-free (BF) operation). The reagent described herein are in one-to-one correspondence with analysis items. That is, specific reagents corresponding to different analysis items are generally different in terms of formula, reagent quantity, component quantity and the like. Depending on the difference in specific analysis items, the reagent usually includes multiple components, for example, usually include 2 to 5 components, including magnetic particles, an enzyme label, a diluent, a dissociation reagent, and the like. For example, a T4 reagent (thyroxine) contains three components: magnetic particles, an enzyme label, and a dissociation agent. According to different reaction modes, multiple reagent components for one analysis item can be dispensed at one time or dispensed in multiple steps. When dispensed in steps, the multiple reagent components are defined as a first reagent, a second reagent, a third reagent, and so on according to the order of dispensation. After the incubation is finished, the BF operation is performed. The BF operation refers to a process of capturing a complex of the bound magnetic particles, antigens and labeled antibodies with a magnetic field, while removing free labeled antibodies and other unreacted or unbound ingredients (for the convenience of description, referred to simply as unbound ingredients herein). After the BF operation is finished, a signal reagent is dispensed for a signal incubation (generally for 1 to 6 minutes). Finally, the amount of luminescence (for the convenience of description, referred to as a reactant signal herein) produced by a reaction between a labeling reagent and the signal reagent is measured. The signal reagent is used to measure the generated signal (usually the amount of luminescence), which is usually a kind of general reagent, and is in one-to-many correspondence with the analysis items. That is, different analysis items share the signal reagent. The signal incubation refers to a process in which the signal reagent is added into the reactor subjected to the BF operation, for reaction for a period of time in a constant temperature environment, to increase the signal. It should be noted that due to the difference in the specific ingredients of the signal reagent, some luminescence systems do not require the signal incubation, and a measurement can be performed directly during or after dispensing the signal reagent. One or more types of signal reagents may be used. For example, some signal reagents include a first signal reagent, a second signal reagent, and so on. In an immunoassay device, subjected to the above processes, the antigens or antibodies contained in a sample bound to the labeling reagent is quantitatively or qualitatively measured. In addition, an immunoassay analyzer can analyze the sample through several different analysis items.

A working period or cycle, abbreviated as a period, is the shortest time window that can be reproduced cyclically during a test, which usually has a fixed length of time. Within the time of a period, a certain number of process operations, tasks, work packages or the like are performed serially or in parallel in a controlled order. For example, the operations and tasks may be liquid pipetting, mixing, incubating, BF operation, measurement, and the like. The tasks of the same component in one period are usually performed serially. The tasks of different components in the same period depend on whether there is a dependency between the actions of related components, and correspondingly, can be performed serially or in parallel. All process operations performed in one period are performed only when required, and not necessarily repeated in the other period. In particular, some process operations may be repeated in each period, while the others may be performed every two or more periods. When multiple tests are performed continuously, since each test is usually at a different stage in a test process, among all the process operations performed in a single period, only some process operations are dedicated to be performed in one test, and some other process operations are performed in other tests. That is, in a single period, different process operations are dedicated to be used in different tests. Therefore, the test is usually completed in multiple periods, where the different process operations used to be performed in the test are performed in different periods. In order to improve test efficiency and throughput, for components with a speed bottleneck, the test may be achieved by increasing the number of components and extending the period of components, such that the working periods of different components are not necessarily the same. That is, there may be multiple parallel periods in the same system. Usually, there is a multiple relation between time lengths of the multiple parallel periods. The multiple is usually equal to the number of the same component. When there are two working periods, they are referred to as a first period and a second period, respectively. For example, when N reagent pipetting units are provided (N≥2, which is a natural number), each reagent pipetting unit works in the first period. The length of the first period is N times the length of the second period. Sequences of actions of the N reagent units are continuously "staggered in parallel" at an interval of the second period. The present application can realize an immune test with a high throughput. The typical length of the second period is 4 to 15 seconds, and the corresponding test throughput is 900 to 240 tests per hour. That is, 900 to 240 results can be continuously reported per hour.

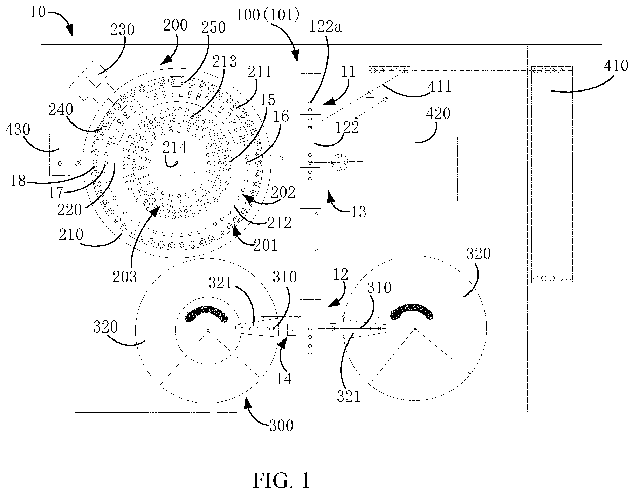

Referring to FIGS. 1 to 4, an immunoassay analyzer 10 according to an embodiment of the present application includes a mixing device 100, a reaction device 200, a reagent supply device 300, a sample supply device 410, and a reactor supply device 420. The reactor supply device 420 is used to provide a clean and empty reactor 20. The sample supply device 410 is used to add a sample into the empty reactor 20. The reagent supply device 300 is used to add a reagent into the reactor 20 containing the sample. The mixing device 100 is used to mix the reactor 20 containing the sample and the reagent. The reaction device 200 is used to incubate, wash, and measure the mixed sample and reagent in the reactor 20.

In some embodiments, the reactor supply device 420 includes a supply silo, a sorting mechanism, a supply chute, and a supply tray. The supply silo is used to store the clean and empty reactor 20. The supply silo can be located behind the reagent supply device 300, such that the whole machine space can be fully utilized, and thus a structure of the immunoassay analyzer 10 becomes more compact. The sorting mechanism is used to sort the randomly placed reactors 20, and arrange them in a certain order. The supply chute guides the sorted reactors 20 into the supply tray one by one. The supply tray is used to temporarily store the reactors 20 conveyed by the supply chute. The reactors 20 can be arranged at intervals along a circumferential direction of the supply tray. The supply tray can rotate around its own central axis to drive the reactor 20 to a designated position. The designated position can be defined as a reactor supply station. The reactor 20 on the supply tray will be transferred to the mixing device 100 at the reactor supply station.

In some embodiments, the sample supply device 410 includes a sample rack, a sample tube, a conveying track, a sample pipetting unit 411, and the like. The sample rack can cooperate with the conveying track. The sample tube is placed on the sample rack. The sample tube is used to contain the sample. For example, about five to ten sample tubes can be placed on each sample rack. When the sample rack drives the sample tube to a designated position along the conveying track, the sample pipetting unit 411 pipets the sample of the sample tube, and adds the sample into the empty reactor 20. The sample pipetting unit 411 can be provided with a steel needle or a disposable suction nozzle. In order to pipet the sample smoothly, the sample pipetting unit 411 can move vertically up and down, move horizontally and linearly, rotate horizontally or the like.

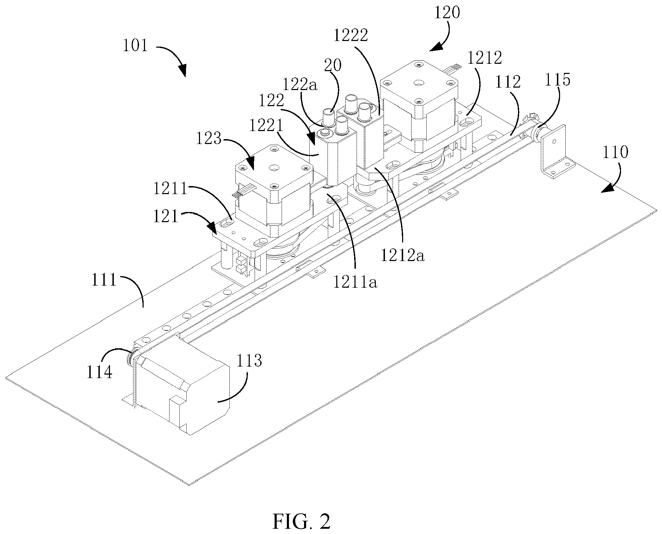

Referring to FIGS. 1 and 2, in some embodiments, the mixing device 100 is of a serial type. The serial type mixing device 101 includes a transport assembly 110 and a mixing assembly 120. The transport assembly 110 is provided with at least two mixing assemblies 120. The transport assembly 110 can drive all the mixing assemblies 120 to move in the same direction synchronously. In short, all the mixing assemblies 120 are connected in series on one transport assembly 110.

The transport assembly 110 includes a frame 111 and a conveyor provided on the frame 111. The conveyor is used to drive all the mixing assemblies 120 to move in the same direction synchronously, which can be composed of one or more of the transmission forms or mechanisms such as a synchronous belt, a screw drive, and a gear rack.

In some embodiments, the conveyor includes a motor 113, a driving wheel 114, a driven wheel 115, and a synchronous belt 112. The motor 113 is used to drive the driving wheel 114 to rotate. The synchronous belt 112 is wound around the driving wheel 114 and the driven wheel 115. When the motor 113 rotates, the driving wheel 114 and the driven wheel 115 drive the synchronous belt 112 to move.

Each mixing assembly 120 includes a support base 121, a driver 123 and a carrying platform 122. The support base 121 is slidably provided on the frame 111 and connected to the conveyor of the transport assembly 110. Specifically, a sliding rail may be provided on the frame 111. The support base 121 cooperates with the sliding rail. The synchronous belt 112 drives the frame to slide in an extending direction of the sliding rail. The driver 123 is mounted on the support base 121 and connected to the carrying platform 122. The reactor 20 is placed on the carrying platform 122. The synchronous belt 112 can drive the support base 121 of each mixing assembly 120 to move in the same direction. The driver 123 can drive the carrying platform 122 to shake eccentrically, such that the reactant in the reactor 20 is mixed due to non-contact eccentric shaking.

The carrying platform 122 may be provided with at least two accommodating holes 122a. The reactors 20 are inserted into the accommodating holes 122a, such that the carrying platform 122 can carry the reactors 20. In other embodiments, the accommodating hole 122a can also be a solid structure such as a bracket, as long as the reactor 20 can be placed on the carrying platform 122.

When two mixing assemblies 120 are provided, one of the mixing assemblies 120 includes a first support base 1211 and a first carrying platform 1221, and the other mixing assembly 120 includes a second support base 1212 and a second carrying platform 1222. The first support base 1211 has a first mounting end 1211a. The second support base 1212 has a second mounting end 1212a. The second mounting end 1212a is provided adjacent to the first mounting end 1211a. The first carrying platform 1221 is located at the first mounting end 1211a. The second carrying platform 1222 is located at the second mounting end 1212a. In short, the first carrying platform 1221 and the second carrying platform 1222 are provided opposite to each other, such that the sample and the reagent can be easily added into the reactor 20 on the different carrying platform 122 at the designated positions.

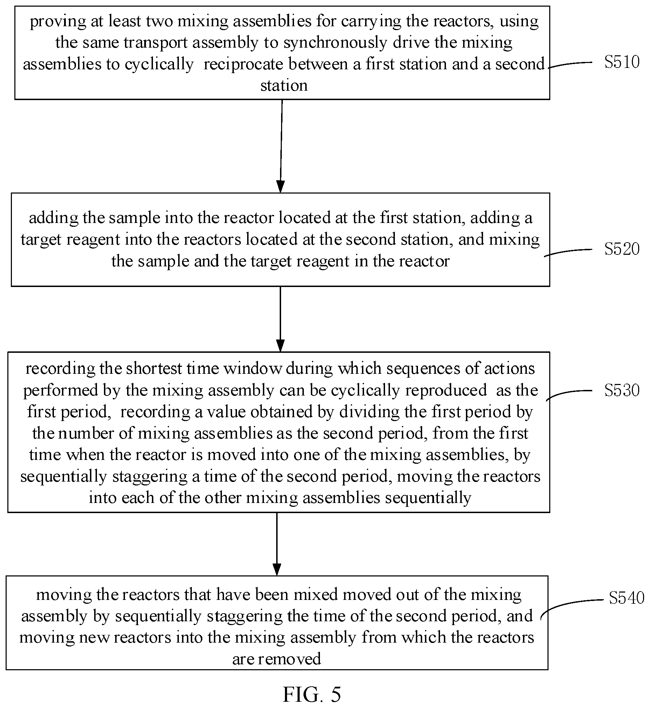

Referring to FIG. 5, when the above serial type mixing device 101 is used to mix the sample and the reagent, a serial type mixing method can be formed. The serial type mixing method mainly includes the following steps.

At S510, at least two mixing assemblies 120 for carrying the reactors 20 are provided. The same transport assembly 110 is used to synchronously drive the mixing assemblies 120 to cyclically reciprocate between a first station 11 and a second station 12. That is, the synchronous belt 112 drives all the carrying platforms 122 to move between the first station 11 and the second station 12.

At S520, the sample is added into the reactor 20 located at the first station 11. The reagent is added into the reactor 20 located at the second station 12. The sample and the reagent in the reactor 20 are mixed. When the synchronous belt 112 drives the carrying platform 122 to move to the first station 11, the synchronous belt 112 stops moving. Since the sample pipetting unit 411 is provided near the first station 11, the sample pipetting unit 411 will pipet the sample and add it to one reactor 20. After the sample is added, when the synchronous belt 112 drives the carrying platforms 122 to move to the second station 12, the synchronous belt 112 stops moving, and the reagent can be added into the reactor 20 containing the sample through the reagent pipetting unit 310 in the reagent supply device 300. When the sample and the reagent are added into the reactor 20, the driver 123 can drive the carrying platform 122 to shake eccentrically, such that the sample and the reagent in the reactor 20 are mixed by non-contact eccentric shaking.

At S530, the shortest time window during which the sequences of actions or tasks performed by the mixing assembly 120 can be cyclically reproduced, including actions of receiving the reactor 20, waiting the sample pipetting unit 411 to add the sample, waiting the reagent pipetting unit 310 to add the reagent, shaking eccentrically, removing the reactor 20 that has been mixed and the like, is recorded as the first period. That is, the minimum time interval at which the mixing assembly 120 performs the same action twice in succession is the first period. A value obtained by dividing the first period by the number of mixing assemblies 120 is recorded as the second period. From the first time when the reactor 20 is moved into one of the mixing assemblies 120, a time of the second period is sequentially staggered, such that the reactors 20 are moved into each of the other mixing assemblies 120 sequentially. It can be understood that, in order to implement the above steps, the working period of the transport assembly 110 is the second period, and the working period of the mixing assembly 120 is the first period. The transport assembly 110 can synchronously drive the mixing assembly 120 to cyclically reciprocate between the first station 11 and the second station 12 in each second period.

At S540, the reactors 20 that have been mixed are moved out of the mixing assembly 120 by sequentially staggering the time of the second period. Then, new reactors 20 are moved into the mixing assembly 120 from which the reactors 20 are removed.

The present application can realize a high throughput immunity test. The length of the second period can be any suitable value within 4 to 15 seconds, such as 4 seconds, 5 seconds, 6 seconds, 9 seconds, or the like, and the corresponding test throughput is 900 to 240 tests per hour. That is, 900 to 240 results can be continuously reported per hour.

In the following description, 10 seconds are taken as an example for the convenience.

The following takes the transport assembly 110 driving the two mixing assemblies 120 to move synchronously as an example. If the immunoassay analyzer 10 is necessary to finish a measurement of one reactor 20 every 10 seconds, that is, reports one test result every 10 seconds, the time of the second period is 10 seconds. Regarding the entire immunoassay analyzer 10 as a pipeline, it is necessary to ensure that the throughput throughout the pipeline is the same. Therefore, the mixing device 100 is also necessary to output one reactor 20 that has been mixed every 10 seconds. If there is only one mixing assembly 120, since a total time required for the sequence of actions performed by the mixing assembly 120, including receiving the reactor 20, waiting the sample pipetting unit 411 to add the sample, waiting the reagent pipetting unit 310 to add the reagent, shaking eccentrically, removing the reactor 20 that has been mixed and the like, is greater than 10 seconds, the mixing device 100 will not be able to output one reactor 20 that has been mixed every 10 seconds. Therefore, the throughput of the mixing device 100 is lower than that of the pipeline, causing the pipeline to fail to work continuously at maximum efficiency. Therefore, the first period is set to be twice the second period, that is, the first period is 20 seconds, and two mixing assemblies 120 are provided. The two mixing assemblies 120 perform the sequences of actions by relatively staggering the time of the second period (namely, 10 seconds), that is, the two mixing assemblies 120 are "staggered in parallel" at an interval of the second period. Based on each mixing assembly 120 outputting one reactor 20 that has been mixed every 20 seconds, the entire mixing device 100 will output one reactor 20 that has been mixed every 10 seconds, finally achieving the purpose of "quantity shortening the time".

In other embodiments, an initial station 13 can further be provided such that the transport assembly 110 drives the mixing assembly 120 to cyclically reciprocate among the initial station 13, the first station 11, and the second station 12. At the initial station 13, the reaction device 20 is moved into or out of the mixing assembly 120. The initial station 13, the first station 11, and the second station 12 can be arranged on the same straight line, and the initial station 13 is located between the first station 11 and the second station 12, such that a moving trajectory of the mixing assembly 120 among the initial station 13, the first station 11, and the second station 12 is a straight line. The initial station 13, the first station 11 and the second station 12 can also be arranged on the same circumference, such that the mixing assembly 120 is moved circularly among the initial station 13, the first station 11 and the second station 12. Compared with a conventional mixing assembly 120 fixed in a single station, the transport assembly 110 of the present application drives the mixing assembly 120 to cyclically reciprocate between multiple stations, such that the mixing assembly 120 can perform different sequences of actions at different stations in an orderly manner, which shortens the moving trajectories of the sample pipetting unit 411, the reagent pipetting unit 310 and the like. Thus, the mixing assembly 120 can achieve more flexible and efficient task operations of the reactor 20, such as receiving the reactor 20, receiving the sample, receiving the reagent, mixing, thereby improving the test throughput of the whole machine.

Specifically, when the serial type mixing device 101 starts to work, one reactor 20 is added to the first carrying platform 1221 at the initial station 13 for the first time. In this case, no reactor 20 is added to the second carrying platform 1222. The conveyor drives the first carrying platform 1221 and the second carrying platform 1222 to move from the initial station 13 to the first station 11, and thus the sample is added into the reactor 20 on the first carrying platform 1221. The conveyor drives the first carrying platform 1221 and the second carrying platform 1222 to move from the first station 11 to the second station 12, and thus the reagent is added into the reactors 20 containing the sample on the first carrying platform 1221. The first carrying platform 1221 generates eccentric shaking, such that the sample and the reagent in the reactor 20 start to be mixed. The conveyor drives the first carrying platform 1221 and the second carrying platform 1222 to return from the second station 12 to the initial station 13, in this case, the first carrying platform 1221 and the second carrying platform 1222 arrive at the initial station 13 at the 10^{th} second, and thus the reactor 20 is added to the second carrying platform 1222 for the first time. The conveyor drives the first carrying platform 1221 and the second carrying platform 1222 to move from the initial station 13 to the first station 11 again, and thus the sample is added into the reactor 20 on the second carrying platform 1222. The conveyor drives the first carrying platform 1221 and the second carrying platform 1222 to move from the first station 11 to the second station 12 again, and thus the reagent is added into the reactor 20 containing the sample on the second carrying platform 1222. The carrying platform 1222 generates eccentric shaking, such that the sample and reagent in the reactor 20 start to be mixed. The conveyor drives the first carrying platform 1221 and the second carrying platform 1222 to return from the second station 12 to the initial station 13, in this case, the first carrying platform 1221 and the second carrying platform 1222 arrive at the initial station 13 at the 20^{th} second. The reactor 20 is added to the first carrying platform 1221 for the second time and is moved to the first station 11. According to this mixing rule, when the first carrying platform 1221 and the second carrying platform 1222 arrive at the initial station 13 at the 20^{th} second, the reactor 20 is added to the second carrying platform 1222 for the second time. As such, when the first carrying platform 1221 and the second carrying platform 1222 arrive at the initial station 13 at the 20^{th}, 30^{th}, and (N0)^{th} second (N≥2), the reactor 20 will be moved into the mixing device 100. Similarly, since the working period of each mixing assembly 120 is the first period (20 seconds) and the sequences of actions between the mixing assemblies 120 are staggered in parallel at the time interval of the second period (10 seconds), one reactor 20 is mixed by each mixing assembly 120 every 20 seconds, and is moved out of the mixing device 100 to the reaction device 200. Nevertheless, the entire mixing device 100 outputs one reactor 20 that has been mixed every 10 seconds, such that the throughput of the mixing device 100 is equal to the throughput of the pipeline. In fact, when the reactor 20 on one of the mixing assemblies 120 is mixed, the mixing time is fully utilized to add the sample or the reagent into the reactor 20 on the other mixing assembly 120 to make the whole the throughput of the mixing device 100 meet the requirements of the test throughput.

In other embodiments, when the second period is still 10 seconds, the time of the first period can be longer. In this case, three, four or more mixing assemblies 120 are provided. The first period can be set to be three times, four times, or even more times the second period. That is, the first period is 30 seconds or 40 seconds, etc. In this way, on the basis of ensuring the test throughput, the moving speed of the transport assembly 110 can be reduced, the mixing time of the sample and the reagent can be prolonged, and thus the bottleneck of the moving speed of the transport assembly 110 and the bottleneck of the mixing time of the sample and the reagent can be effectively solved. In the case that the moving speed of the transport assembly 110 and the mixing time of the sample and the reagent are fixed, each mixing assembly 120 still outputs one reactor 20 that has been mixed every 20 seconds. That is, the first period is still 20 seconds. If it is necessary to increase the test throughput of the immunoassay analyzer 10, for example, it is required to output one reactor 20 after the measurement is completed every 5 seconds (the second period), the number of mixing assemblies 120 on the transport assembly 110 can be increased to four. For another example, if it is required to output one reactor 20 after the measurement is completed every 4 seconds (the second period), the number of mixing assemblies 120 on the transport assembly 110 can be increased to five.

At least two mixing positions can be provided on each mixing assembly 120. The mixing position is the accommodating hole 122a on the carrying platform 122. When one of the mixing positions (the accommodating hole 122a) is occupied by one reactor 20 that is being mixed or has been mixed, another reactor 20 is moved into the other empty mixing position (accommodating hole 122a) on this mixing assembly 120. This can solve the occupying problem of the mixing positions during the reactor 20 being moving into and out of the same carrying platform 122, and improve the test efficiency and test throughput.

The mixing of the sample and the reagent in the reactor 20 can be performed after a movement of the mixing assembly 120 driven by the transport assembly 110 is stopped, or during the movement. For example, during the mixing assembly 120 returning from the second station 12 to the initial station 13, the driver 123 causes the carrying platform 122 to eccentrically shake to mix the sample and the reagent. The mixing during the movement can make full use of the time during the movement of the mixing assembly 120, to mix the sample and the reagent, ensuring that the mixing device 100 meets the test throughput requirements.

From the start of mixing to the completion of mixing, the time required by the reactor 20 loaded with the sample and the reagent is usually 2 to 10 seconds. The working period of the transport assembly 110 is the first period, and the working periods of the two mixing assemblies 120 are the second period, such that there is enough time for the sample and the reagent to be mixed to ensure that the samples and the reagent can react with each other sufficiently and improve the accuracy of the subsequent measurement.

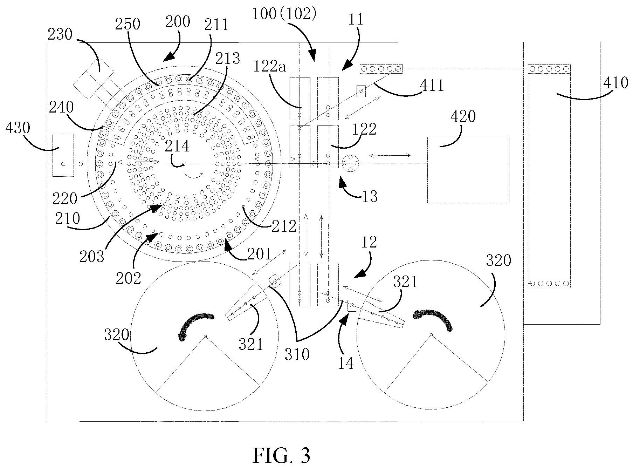

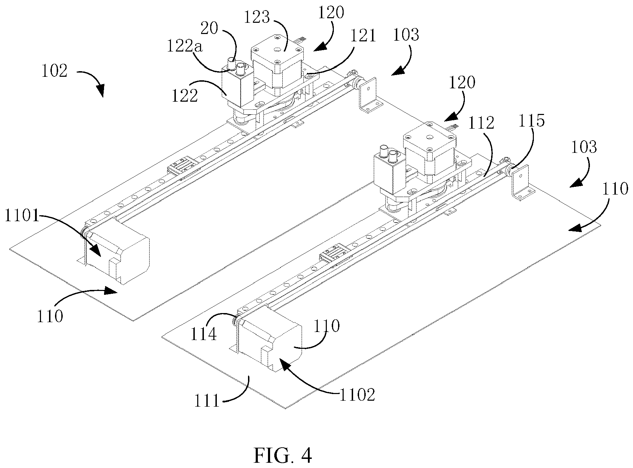

Referring to FIGS. 3 and 4, in some embodiments, the mixing device 100 is of a parallel type. The parallel type mixing device 102 includes at least two mixing mechanisms 103. Each mixing mechanism 103 includes a transport assembly 110 and a mixing assembly 120. The mixing assembly 120 is provided on the transport assembly 110. The transport assembly 110 drives the mixing assembly 120 to move. For example, when each mixing mechanism 103 includes one transport assembly 110 and one mixing assembly 120, the displacements of the mixing assemblies 120 are in parallel with each other. Structures of the transport assembly 110 and the mixing assembly 120 are the same as the corresponding structures in the above-mentioned serial type mixing device 100. That is, each transport assembly 110 includes a frame 111 and a conveyor provided on the frame 111. Each mixing assembly 120 includes a support base 121, a driver 123 and a carrying platform 122, which will not be repeated here. The main difference between the parallel type mixing device 102 and the serial type mixing device 100 is that the mixing assemblies 120 are respectively provided on different transport assemblies 110, and the movements of the mixing assemblies 120 on the different transport assemblies 110 are not synchronized.

In some embodiments, at least one mixing mechanism 103 includes one transport assembly 110 and at least two mixing assemblies 120. The transport assembly 110 drives the at least two mixing assemblies 120 to move synchronously. In this case, the at least two mixing assemblies 120 on the mixing mechanism 103 are in series. The mixing assemblies 120 on such mixing mechanism 103 are in parallel with the mixing assemblies 120 on the other mixing mechanism(s) 103. That is, the mixing assemblies 120 in the entire mixing device 100 are in parallel and in series, that is, in hybrid.

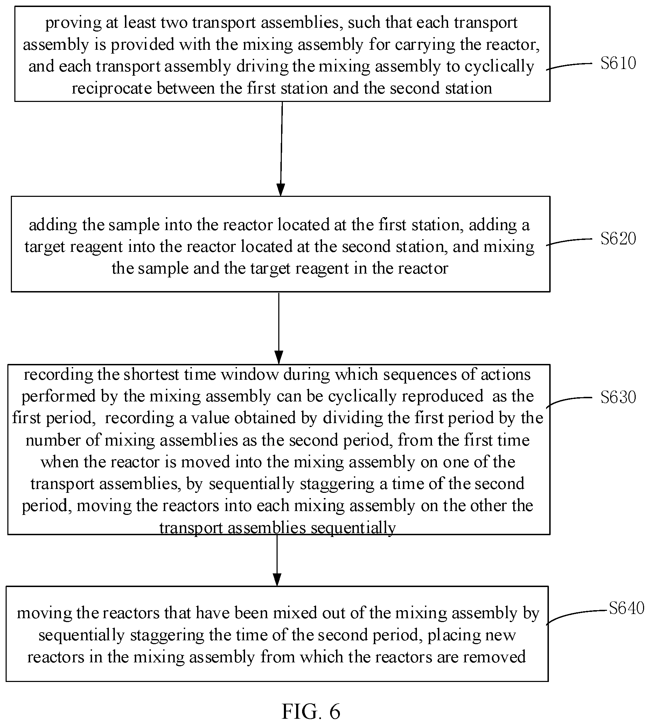

Referring to FIG. 6, when the above-mentioned parallel type mixing device 102 is used to mix the sample and the reagent, a parallel type mixing method can be formed. The parallel type mixing method mainly includes the following steps.

At S610, at least two transport assemblies 110 are provided, such that each transport assembly 110 is provided with the mixing assembly 120 for carrying the reactor 20, and each transport assembly 110 drives the mixing assembly 120 to cyclically reciprocate between the first station 11 and the second station 12.

At S620, the sample is added into the reactor 20 located at the first station 11, and then the reagent is added into the reactor 20 located at the second station 12, and then the sample and the reagent in the reactor 20 are mixed.

At S630, the shortest time window during which the sequences of actions or tasks performed by the mixing assembly 120 can be cyclically reproduced, including actions of receiving the reactor 20, waiting the sample pipetting unit 411 to add the sample, waiting the reagent pipetting unit 310 to add the reagent, shaking eccentrically, removing the reactor 20 that has been mixed and the like, is recorded as the first period. That is, the minimum time interval at which the mixing assembly 120 performs the same action twice in succession is the first period. A value obtained by dividing the first period by the number of mixing assemblies 120 is recorded as the second period. From the first time when the reactor 20 is moved into the mixing assembly 120 on one of the transport assemblies 110, the time of the second period is sequentially staggered, such that the reactors 20 are moved into other mixing assembly 120 on the other transport assemblies 110 sequentially. It can be understood that, in order to implement the above steps, the working periods of each transport assembly 110 and each mixing assembly 120 are the second period.

At S640, the reactors 20 that have been mixed are moved out of the mixing assembly 120 by sequentially staggering the time of the second period. Then, new reactors 20 are placed in the mixing assembly 120 from which the reactors 20 are removed.

In the following, two transport assemblies 110 are provided. Each transport assembly 110 provided with one mixing assembly 120 is taken as an example for description. For the same portions as the serial type mixing method, please refer to the above description. Assuming that the second period is 10 seconds, and each mixing mechanism 103 outputs one reactor 20 that has been mixed every 20 seconds, that is, the first period is 20 seconds, because the reactors 20 are moved into the mixing assemblies 120 on the other transport assemblies 110 sequentially by sequentially staggering the time of the second period, and finally, the entire mixing device 100 will output one reactor 20 that has been mixed every 10 seconds, which can function as "quantity shortening the time" as well.

Referring to the above description of the serial type mixing method, in the parallel type mixing method, the initial station 13 can also be provided, such that the transport assembly 110 drives the mixing assembly 120 to cyclically reciprocate among the initial station 13, the first station 11, and the second station 12. At the initial station 13, the reactor 20 is moved into or out of the mixing assembly 120. The initial station 13, the first station 11 and the second station 12 can be arranged on the same straight line, and the initial station 13 is located between the first station 11 and the second station 12, such that a moving trajectory of the mixing assembly 120 among the initial station 13, the first station 11 and the second station 12 is a straight line.

Compared with a conventional mixing assembly 120 fixed in a single station, the transport assembly 110 of the present application drives the mixing assembly 120 to cyclically reciprocate between multiple stations, which improves the test throughput of the whole machine.

Each mixing assembly 120 is provided with at least two mixing positions. The mixing position is the accommodating hole 122a on the carrying platform 122. The two mixing positions are used simultaneously or alternately, which can improve the efficiency of the mixing assembly 120 mixing the reactor 20. When one of the mixing positions (accommodating hole 122a) is occupied, the reactor 20 can be moved to the other mixing position (accommodating hole 122a) on the same mixing assembly 120. The sample and the reagent in the reactor 20 can be mixed during the movement of the mixing assembly 120 driven by the transport assembly 110 or after the movement is stopped. That is, the mixing of the sample and the reagent in the reactor 20 is not limited by the moving state of the transport assembly 110, which can make the mixing device 100 more flexible and efficient.

Specifically, when the parallel type mixing device 100 starts to work, one of the transport assemblies 110 is recorded as a first transport assembly 1101, and the other transport assembly 110 is recorded as a second transport assembly 1102. One reactor 20 is added into the carrying platform 122 on the first transport assembly 1101 at the initial station 13 for the first time, while no reactor 20 is added into the carrying platform 122 on the second transport assembly 1102.

For the first transport assembly 1101, when it moves from the initial station 13 to the first station 11, the sample is added into the reactor 20 on the carrying platform 122 of the first transport assembly 1101. When the first transport assembly 1101 moves from the first station 11 to the second station 12, and the reagent is added into the reactor 20 containing the sample on the carrying platform 122 of the first transport assembly 1101. The carrying platform 122 of the first transport assembly 1101 generates eccentric shaking such that the sample and the reagent in the reactor 20 start to be mixed.

For the second transport assembly 1102, at the 10^{th} second after adding the reactor 20 into the carrying platform 122 on the first transport assembly 1101, the reactor 20 is added into the carrying platform 122 on the second transport assembly 1102 for the first time, and the second transport assembly 1102 starts to move regularly according to the moving rule of the first transport assembly 1101. As such, when the carrying platform 122 on the first transport assembly 1101 and the carrying platform 122 on the second transport assembly 1102 arrive at the initial station 13 at the 20^{th}, 30^{th}, and (n0)^{th} second, the reactor 20 will be moved into the mixing device 100. Similarly, since the working period of each mixing assembly 120 is the first period (20 seconds) and the sequences of actions between the mixing assemblies 120 are staggered in parallel at the time interval of the second period (10 seconds), one reactor 20 is mixed by each mixing assembly 120 every 20 seconds, and is moved out of the mixing device 100 to the reaction device 200. Nevertheless, the entire mixing device 100 outputs one reactor 20 that has been mixed every 10 seconds.

For the parallel type mixing method, the transport assemblies 110 drive the mixing assemblies 120 to be "staggered in parallel" at the interval of the second period (10 seconds). Although each mixing mechanism 103 outputs one reactor 20 that has been mixed every 20 seconds (first period), the two mixing mechanisms 103 starts to work from the initial station 13 by being staggered 10 seconds, such that the entire mixing device 100 outputs one reactor 20 that has been mixed every 10 seconds (the second period). By increasing the number of mixing mechanisms 103, on the basis of the throughput of the entire mixing device 100 meeting the test throughput requirements, the transport assemblies 110 can move at a slower speed, which solves the bottleneck of the moving speed of the transport assembly 110 and the bottleneck of the mixing time of the sample and the reagent. For other similarities, please refer to the relevant description of the above serial type mixing method.

When at least one transport assembly 110 is provided with at least two mixing assemblies 120 thereon, the transport assembly 110 drives all the mixing assemblies 120 provided thereon to move synchronously. That is, at least one mixing mechanism 103 includes at least two mixing assemblies 120. The mixing assemblies 120 on the mixing mechanism 103 are connected in series. Therefore, the mixing assemblies 120 on the entire mixing device 100 are both connected in parallel and in series. Likewise, the reactor 20 is added to each mixing assembly 120 for the first time at the interval of the second period, and finally the entire mixing device 100 outputs one reactor 20 that has been mixed at the interval of the second period. By providing some of the mixing assemblies 120 to be connected in series, the structure of the entire mixing device 100 can be made more compact.

Referring to FIGS. 1 and 3, in some embodiments, the reagent supply device 300 is provided adjacent to the second station 12. The reagent supply device 300 includes the reagent pipetting unit 310 and a storage unit 320. At least two storage units 320 are provided. The storage unit 320 is provided with a plurality of storage portions 321. The storage portion 321 is used to be place with and store a reagent container. The reagent is contained in the reagent container. The reagent pipetting unit 310 is used to pipet the reagent component in the reagent container on the storage portion 321, and adds the reagent component into the reactor 20 located at the second station 12. The number of storage portions 321 can be set according to needs. Considering the use requirements, cost, and layout, 15 to 50 storage portions 321 are preferably provided on each storage unit 320. For example, 25 storage portions 321 are provided on each storage unit 320. In this way, two storage units 320 can store 50 reagent containers in total in situ at the same time. Each storage unit 320 stores all the reagent components required by the corresponding analysis item. For example, in an analysis project, it is necessary to add three reagent components that are the magnetic particles, the enzyme label, and the dissociating agent into the reactor 20, and thus the three reagent components of the magnetic particles, the enzyme label, and the dissociating agent are stored in the same storage unit 320. When a certain analysis item needs to load multiple reagent containers to expand the test throughput on machine of this item, the multiple reagent containers can be stored in each storage unit in any suitable combination. For example, when two storage units are provided, it is necessary to load three thyroid stimulating hormone (TSH) reagent containers, each for 100 tests. The three TSH reagent containers can be loaded in the same storage unit, or one TSH reagent container is loaded in one storage unit, and the other two TSH reagent containers are loaded in the other storage unit.

For a conventional reagent supply device 300, in order to increase the storage capacity for the analysis items, the number of storage portions 321 is necessary to be increased, which causes an increase in a size of the entire storage unit 320. It is disadvantageous to the layout and the manufacturing of the storage unit 320 when the storage unit 320 occupies a large area. On the other hand, for the storage unit 320 with a large volume and weight, the difficulty of controlling the movement of the storage unit 320 is also increased, resulting in the storage portion 321 being unable to arrive at the designated position in a short time for the reagent pipetting unit 310 to pipet the reagent, which becomes a bottleneck for achieving high test throughput. In addition, the conventional reagent supply device 300 stores multiple reagent components for the same analysis item on different storage units 320, which not only allows the reagent pipetting unit 310 to pipet the reagent of the same analysis item on multiple different storage units 320, resulting in the reagent pipetting unit 310 having a large displacement and a complex moving logic, and being unable to achieve high test throughput, but also requires the reagent components to be contained in multiple reagent containers, resulting in high manufacturing costs and inconvenient operations for users. In addition, since multiple reagent components of the same analysis item are stored in different storage units 320, when a certain storage unit fails, it will directly cause the apparatus to fail to continue testing.

According to the above embodiment of the present application, the reagent supply device 300 is provided with at least two storage units 320, each of which has a small volume, which is advantageous to the overall machine layout and the movement control, and can further ensure that the entire reagent supply device 300 has a large reagent storage capacity. In addition, each storage unit 320 stores all the reagent components required by the corresponding analysis item, which can improve the reliability of the reagent supply device 300 and the tolerance to failure. When one of the storage units 320 fails and cannot work, the remaining storage unit 320 can continue to work to ensure that the reagent supply device 300 can still work effectively. In other embodiments, the failed storage unit 320 can be maintained while other storage units 320 are working.

The storage unit 320 may be a rotating disk. The rotating disk rotates periodically and intermittently, thereby driving the storage portion 321 to the designated position (i.e., a pipetting station 14), such that the reagent pipetting unit 310 pipets the reagent on the storage portion 321 at the pipetting station 14. The number of reagent pipetting units 310 can be equal to the number of rotating disks. Each rotating disk corresponds to one reagent pipetting unit 310. Each reagent pipetting unit 310 pipets the reagent from the corresponding rotating disk. As similar to the sample pipetting unit 411, the reagent pipetting unit 310 can use a steel needle or a disposable suction nozzle. In order to pipet the sample smoothly, the reagent pipetting unit 310 can move vertically up and down, move horizontally and linearly, rotate horizontally or the like. In other embodiments, one reagent pipetting unit 310 can also be provided, and the one reagent pipetting unit 310 pipets the reagents in multiple rotating disks.

The reagent supply device 300 further includes a scanner. The scanner is provided on the storage unit 320. The scanner can identify barcode information of the reagent container on the storage portion 321, so as to distinguish different reagents. In order to make the structure of the entire reagent supply device 300 compact, the scanner is fixed. The storage unit 320 can further be provided with a refrigerator. The refrigerator can refrigerate the reagent in the storage portion 321, thereby storing the reagent in situ for a long term.

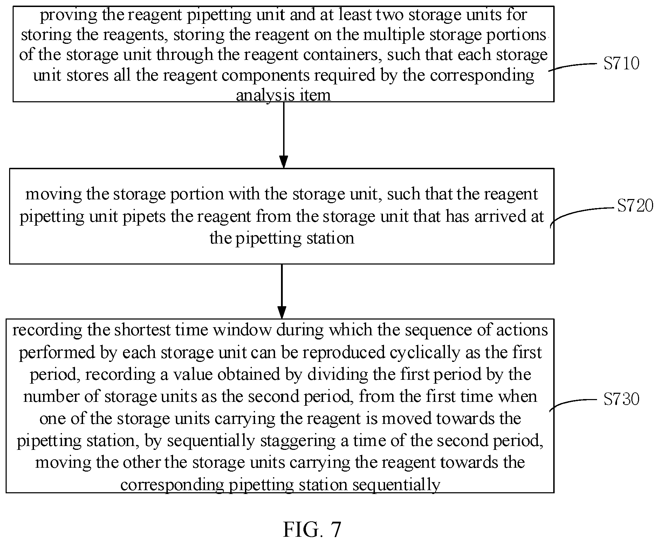

Referring to FIG. 7, in order to achieve a high throughput immune test, when the reagent supply device 300 is used to pipet the reagent, a pipetting method of the reagent can be formed, and the pipetting method mainly includes the following steps.

At S710, the reagent pipetting unit 310 and at least two storage units 320 for storing the reagent are provided. The reagent containers are stored on the multiple storage portions 321 of the storage unit 320, such that each storage unit 320 store all the reagent components required by the corresponding analysis item.

At S720, the storage portion 321 is moved along with the storage unit 320, such that the reagent pipetting unit 310 pipets the reagent from the storage portion 321 that has arrived at the pipetting station 14. The movement of the storage unit 320 may be rotation. For example, the storage unit 320 rotates periodically and intermittently, such that the storage portion 321 arrives at the pipetting station 14 every set time, for the reagent pipetting unit 310 to pipet the reagent.

At S730, the shortest time window during which the sequence of actions performed by each storage unit 320 can be reproduced cyclically is recorded as the first period. That is, the minimum time interval at which the storage unit 320 performs the same action twice in succession is the first period. A value obtained by dividing the first period by the number of storage units 320 is recorded as the second period. From the first time when one of the storage units 320 drives the storage portion 321 to move towards the pipetting station 14, the time of the second period is sequentially staggered, such that the other storage units 320 drive the storage portions 321 towards the corresponding pipetting station 14 sequentially.

Taking two storage units 320 as an example for illustration, according to the principle that the throughput is equal everywhere, the value of the second period is equal to the value of the second period mentioned in the above-mentioned mixing method. Also taking 10 seconds as an example for illustration, that is, every 10 seconds, one storage unit 321 arrives at the pipetting station 14 for the reagent pipetting unit 310 to pipet the reagent. In other embodiments, the value of the first period is the same as the value of the first period mentioned in the above-mentioned mixing method. That is, the value of the first period is 20 seconds. With reference to the basic principle of the above-mentioned mixing method, the two storage units 320 are "staggered in parallel" at a time interval of the second period. Although each storage unit 320 will have one storage unit 321 arrive at the corresponding pipetting station 14 every 20 seconds, the sequences of actions of the two storage units 320 start to be performed by being staggered 10 seconds sequentially, such that the entire reagent supply device 300 will have one storage portion 321 arrive at the pipetting station 14 for the reagent pipetting unit 310 to pipet the reagent every 10 seconds. By increasing the number of storage units 320 to shorten the time, on the basis that the throughput of the entire reagent supply device 300 meeting the test throughput requirements, the storage unit 320 can be rotated at a slower speed, thereby solving the bottleneck of the moving speed of the storage unit. in other embodiments, when the number of storage units 320 increases, the throughput of the entire reagent supply device 300 can be increased without changing the rotating speed of the storage unit 320, thereby increasing the test throughput of the immunoassay analyzer 10.

In some embodiments, if the test throughput of the immunoassay analyzer is appropriately reduced or other high-cost designs are adopted to increase the moving speed of the storage unit, when the moving speed of the storage unit 320 does not constitute a bottleneck for the test throughput of the immunoassay analyzer, the sequences of actions of the multiple storage units 320 can be "in series synchronously". That is, the sequences of actions of the multiple storage units 320 are synchronized in the working period, and are in series during the working period. Each storage unit 320 can position the target storage portion 321 to the pipetting station 14 in each working period for the reagent pipetting unit 310 to pipet the reagent. However, only one storage unit 320 is required to position the target storage portion 321 to the pipetting station 14 in each working period for the reagent pipetting unit 310 to pipet the reagent. Take providing two storage units 320 (denoted as a first storage unit and a second storage unit, respectively) and the working period being 10 seconds as an example for illustration. At the first 10^{th} second, one storage portion 321 of the first storage unit arrives at the pipetting station 14 for the reagent pipetting unit 310 to pipet the reagent. At the second 10^{th} second, one storage portion 321 of the second storage unit arrives at the pipetting station 14 for the reagent pipetting unit 310 to pipet the reagent. At the third 10^{th} second, one storage portion 321 of the first storage unit arrives at the pipetting station 14 for the reagent pipetting unit 310 to pipet the reagent. According to this rule, every 10 seconds, two storage units 320 alternate in series between the periods, and have one storage unit 321 arrive at the pipetting station 14 for the reagent pipetting unit 310 to pipet the reagent. In other embodiments, at the first, second, ... N^{th} 10 second (N≥1), the first storage unit positions the storage portion 321 to the pipetting station 14 for the reagent pipetting unit 310 to pipet the reagent. At the N^{th}, (N+1)^{th}, ... (N+M)^{th} 10 second (M≥1), the second storage unit positions the storage portion 321 to the pipetting station 14 for the reagent pipetting unit 310 to pipet the reagent. In short, in any work period, one of the storage units can position the storage portion 321 to the pipetting station 14 for the reagent pipetting unit 310 to pipet the reagent.

The same storage unit 320 stores all the reagent components required by a test corresponding to an analysis item, such that the reagent pipetting unit 310 can quickly pipet the reagent and increase the throughput of the reagent supplied by the reagent supply device 300. On the other hand, when the storage unit 320 malfunctions, the apparatus can use other storage units 320 to continue the test, such that the apparatus can perform the test normally without affection, which improves the tolerance to failure. In addition, all the reagent components required by a test corresponding to an analysis item are placed in the same storage unit 320, and can be contained in one reagent container with multiple reagent chambers, which not only saves production and manufacturing costs, but also facilitates user operations such as loading and unloading.

During the rotation (revolution) of the storage portion 321 with the storage unit 320, at least one chamber of the reagent container on the storage portion 321 (such as a magnetic particle chamber containing the magnetic particle reagent component) rotates around its own central axis, causing the magnetic particle reagent component in a form of solid suspension to generate a vortex, thereby avoiding the sedimentation of the solid matter (such as the magnetic particles).

The multiple storage units 320 are independently configured. That is, each storage unit 320 can independently rotate to position the reagent on the storage portion 321 to the pipetting station 14. It should be noted that the "independent configuration" used herein has nothing to do with the spatial layout and physical location between the storage units 320. For example, the multiple storage units 320 can be distributed on the apparatus separately without overlapping, or one of the storage units 320 can be embedded at a periphery or an inner side of another storage unit 320. In other embodiments, for better layout and control, the multiple storage units 320 are preferably of the same structure and deployed separately. The multiple storage units 320 are independently configured, which can improve the flexibility of the control, further improve the supply efficiency of the reagent, thereby increasing the processing throughput of the apparatus.

The number of reagent pipetting units 310 may be equal to the number of storage units 320. Each storage unit 320 corresponds to one reagent pipetting unit 310. That is, each storage unit 320 is provided for the reagent pipetting unit 310 corresponding thereto to pipet the reagent. Apparently, when the number of reagent pipetting units 310 is increased, on the basis of meeting the test throughput, the moving speed of each reagent pipetting unit 310 can be reduced, and the bottleneck of the moving speed of the reagent pipetting unit 310 can be solved.

Referring to FIGS. 1 and 3, the reaction device 200 includes a rotating disk 210, a transferring assembly 220, a measuring device 230, and a washing assembly 250. The rotating disk 210 is provided with an incubation ring 203, a washing ring 202 and a measuring ring 201. The incubation ring 203, the washing ring 202, and the measuring ring 201 are all arranged around a rotation center of the rotating disk 210. The incubation ring 203 is provided with incubation positions 213. The incubation positions 213 are arranged at intervals along a circumferential direction of the incubation ring 203. The washing ring 202 is provided with washing positions 212. The washing positions 212 are arranged at intervals along a circumferential direction of the washing ring 202. The measuring ring 201 is provided with measuring positions 211. The measuring positions 211 are arranged at intervals along a circumferential direction of the measuring ring 201. The incubation position 213, the washing position 212, and the measuring position 211 are all used to receive the reactor 20, and may be grooves or brackets, and the like., suitable for carrying the reactor 20. The measuring device 230 is connected to the rotating disk 210. The measuring device 230 can measure an optical signal of the reactor 20 after adding the signal reagent, so as to further analyze the reactant. The washing assembly 250 is located above the washing ring 202, and includes a liquid dispensing portion and a liquid pipetting portion. The liquid dispensing portion dispenses a wash buffer solution into the reactor 20 on the washing position 212. The liquid pipetting portion can be lowered into and raised out of the reactor 20 on the washing position 212, to draw the unbound ingredients in the reactor 20. Further, in order to simplify the structure, the washing assembly 250 further includes a signal reagent dispensing portion configured to dispensing the signal reagent into the reactor 20 subjected to the BF operation on the washing position 212. In some embodiments, the reaction device 200 further includes a waste liquid pipetting assembly 240 and a signal reagent mixing unit 430. The waste liquid pipetting assembly 240 is located above the measuring ring 201. After the measurement of the reactor 20 is finished, the waste liquid pipetting assembly 240 can be lowered into and raised out of the reactor 20 on the measuring position 211, to draw and remove waste liquid in the reactor 20 (mainly the signal reagent), and finally transfer the reactor 20 from which the waste liquid has been drawn and removed to a disposal station, to separate solid waste and liquid waste, thereby reducing the risk of biological hazards. Further, the waste liquid pipetting assembly 240 can be connected to the liquid pipetting portion of the washing assembly 250, and can be lowered to a bottom of the reactor together with the liquid pipetting portion of the washing assembly 250, and then raised out of the reactor after finishing the pipetting. In this way, the function of the washing assembly 250 can be fully utilized, the volume of the mechanism is reduced, the cost is saved, and the problems of complicated structure, high cost and the like caused by the independent provision of the waste liquid pipetting assembly are avoided. The signal reagent mixing unit 430 is provided independently of the rotating disk 210, and includes a mixing assembly similar to or same as the above-mentioned mixing assembly 120, and mixes the reactor 20 containing the signal reagent through the eccentric shaking.

The transferring assembly 220 moves the reactor 20 that has been mixed out of the mixing device 100 and moves it into the incubation position 213. During the reactor 20 rotating with the rotating disk 210, the incubation position 213 incubates the mixed sample and reagent in the reactor 20 for a set time. After finishing the incubation of the reactor 20, the transferring assembly 220 transfers the reactor 20 from the incubation position 213 to the washing position 212. During the reactor 20 rotating with the rotating disk 210, the liquid dispensing portion of the washing assembly 250 can first dispense the wash solution into the reactor 20 at the washing position 212, and then adsorb the magnetic particle composite onto an inner side wall of the reactor 20 through a magnetic field. Then, the liquid pipetting portion of the washing assembly 250 draws the unbound ingredients from the reactor 20. After multiple rounds of "dispensing the washing solution-adsorption-drawing the unbound ingredients", the reactant in the reactor 20 are subjected to the BF operation. After fishing the BF operation of the reactant of the reactor 20, the signal reagent dispensing portion can dispense the signal reagent into the reactor 20. The transferring assembly 220 transfers the reactor 20 after adding the signal reagent from the washing position 212 to the signal reagent mixing unit 430. Then, the reactor 20 is mixed by the signal reagent mixing unit 430. In order to fully mix the signal reagent without affecting the test throughput of the apparatus, the mixing time of the signal reagent is 2 to 6 seconds. After the mixing of the reactor 20 containing the signal reagent is finished, the transferring assembly 220 transfers the reactor 20 from the signal reagent mixing unit 430 to the measuring position 211. If it is necessary to continue the signal incubation of the reactor 20 containing the signal reagent, the measuring position 211 can incubate the reactor 20 for a set time during the reactor 20 rotating with the rotating disk 210. When the reactor 20 advances to the position of the measuring device 230 with the rotating disk 210, the measuring device 230 measures the reactant signal in the reactor 20 to analyze the reactant.

The incubation ring 203, the washing ring 202, and the measuring ring 201 are arranged concentrically. That is, the incubation ring 203, the washing ring 202, and the measuring ring 201 are all centered on the rotation center of the rotating disk 210. The incubation ring 203, the washing ring 202 and the measuring ring 201 are arranged at intervals from inside to outside around the rotation center. That is, the measuring ring 201 is adjacent to an edge of the rotating disk 210. The incubation ring 203 is adjacent to the center of the rotating disk 210. The washing ring 202 is provided between the incubation ring 203 and measuring ring 201. In order to meet the requirements of the incubation time of the analysis item, while ensuring the number of incubation positions 213 without causing the size of the rotating disk 210 of the reaction device 200 to be too large, at least two incubation rings 203 are provided. For example, two to ten incubation rings 203 may be provided. The incubation ring 203 closest to the rotation center is recorded as an inner incubation ring. The incubation ring 203 farthest from the rotation center is recorded as an outer incubation ring. According to the requirement of washing efficiency, one to two washing rings 202 are provided. When one measuring ring 201 is provided, the requirements of the measurement can be met.

The reaction device 200 is provided with an incubation in-out station 15, a washing moving-in station 16, a washing moving-out station 17, and a measuring in-out station 18. In order that the reactor can be moved into and out of each incubation ring 203, washing ring 202, and measuring ring 201 of the reaction device 200, the number of incubation in-out stations 15 is not less than the number of incubation ring 203, and the number of washing moving-in stations 16 and the number of washing moving-out stations 17 are equal to the number of washing rings 202, respectively, and the number of measuring in-out stations 18 is not less than the number of measuring rings 201, that is, being at least one. Further, in order to make the layout of the whole machine compact, while reducing the moving trajectory of the transferring assembly 220 and improving the reliability thereof, and further improving work efficiency, the washing moving-in station 16 and the washing moving-out station 17 are respectively provided at two sides of the rotation center of the rotating disk 210, that is, located at two ends of the washing ring 202 in a dimeter direction thereof. The incubation in-out station 15 is on the same side as the washing moving-in station 16, and the measuring in-out station 18 is on the same side as the washing moving-out station 17. In this way, the reactor that has been moved out of the incubation in-out station 15 can be moved into the washing ring 202 from the washing moving-in station 16 nearby, and the reactor that has been moved out of the washing moving-out station 17 can be moved into the measuring ring 201 from the measuring in-out station 18 nearby.

Specifically, taking a test in a one-step reaction mode an example, the transferring assembly 220 moves the reactor 20 on the mixing device 100 from the incubation in-out station 15 to the incubation position 213. When the reactor 20 is moved to the incubation in-out station 15 with the rotating disk 210, the transferring assembly 220 moves the reactor 20 out of the incubation position 213 from the incubation in-out station 15, and moves the reactor 20 from the washing moving-in station 16 to the washing position 212. When the reactor 20 is moved to the washing moving-out station 17 with the rotating disk 210, the transferring assembly 220 moves the reactor 20 out of the washing position 212 from the washing moving-out station 17, and into the signal reagent mixing unit 430 for signal reagent mixing. After the mixing is finished, the reactor 20 is moved into the measuring position 211 from the measuring in-out station 18. When the reactor 20 is moved to the position of the measuring device 230 with the rotating disk 210, after the measuring device 230 finishes the measurement of the response signal, the reactor 20 continues to move to the position of the waste liquid pipetting assembly 240 with the rotating disk 210. The waste liquid pipetting assembly 240 will draw and remove all the waste liquid in the reactor 20. The reactor 20 from which the waste liquid has been drawn and removed continues to move to the measuring in-out station 18 with the rotating disk 210. In this case, the transferring assembly 220 moves the reactor 20 from which the waste liquid has been drawn after the measurement is finished, out of the measuring position 211 at the measuring in-out station 18, and moves it into a disposal station. When performing tests in other reaction modes, such as delayed one-step or two-step test, the transferring assembly 220 can move the reactor 20 out of the incubation position 213 from the incubation in-out station 15, and move the reactor 20 that has been moved out of the washing position 212 from the washing moving-out station 17 into the mixing device 100.

The moving trajectory of the transferring assembly 220 among the initial station 13, the incubation in-out station 15, the washing moving-in station 16, the washing moving-out station 17, and the measuring in-out station 18 is a straight line. An orthographic projection of the straight line on the rotating disk 210 passes through the rotation center of the rotating disk 210. This simplifies the movement of the transferring assembly 220 and improves the work efficiency of the transferring assembly 220 to meet the requirements of the test throughput. The straight line on which the moving trajectory of the transferring assembly 220 is located further passes through the signal reagent mixing unit 430. The transferring assembly 220 can transfer the reactor 20 among the signal reagent mixing unit 430, the measuring ring 201, and the washing ring 202.

In order to reduce the moving displacement of a single transferring assembly 220 and further improve the work efficiency and control accuracy, two transferring assemblies 220 may be provided, and a relay station 214 is provided at an inner side of the inner incubation ring of the rotating disk 210 (closest to the rotation center). The relay station 214 is used to temporarily carry the reactor 20. The moving trajectory of one of the transferring assemblies 220 on the rotating disk 210 forms a first projection, and the moving trajectory of the other transferring assembly 220 on the rotating disk 210 forms a second projection. The first projection and the second projection are connected into the same straight line at the relay station 214, which is recorded as a trajectory straight line. A straight line that passes through the relay station 214 and is perpendicular to the trajectory straight line is taken as a reference straight line. One of the transferring assemblies 220 is responsible for the transfer of the part of the reactor 20 on the right of the reference straight line, and the other transferring assembly 220 is responsible for the transfer of the part of the reactor 20 on the left of the reference straight line. For example, during the test in a two-step reaction mode, the transferring assembly 220 moves the reactor 20 out of the washing position 212 from the washing moving-out station 17, and into the mixing assembly 120 to add a second reagent, the reactor 20 is required to be transferred from the left part to the right part of the reference straight line. Firstly, the reactor 20 can be transferred from the washing position 212 on the left part of the reference straight line to the relay station through one transferring assembly 220. Then, the reactor 20 can be transferred from the relay station through the other transferring assembly 220 to the mixing unit 120 on the right side of the reference line.

In some embodiments, in order to achieve a compact layout and further improve the efficiency of coordination and cooperation between the transferring assemblies 220, thereby increasing apparatus throughput, the relay station 214 is provided at the rotation center of the rotating disk 210.

Referring to FIGS. 1 and 3, in the immunoassay analyzer 10, the transport assembly 110, the mixing assembly 120, the sample pipetting unit 411, and the reagent pipetting unit 310 can be combined to form a diluting device. That is, the diluting device includes the transport assembly 110, the mixing assembly 120, and the pipetting assembly. The pipetting assembly includes the sample pipetting unit 411 and the reagent pipetting unit 310. In other embodiments, the structures, and positions of the transport assembly 110, the mixing assembly 120, the sample pipetting unit 411, and the reagent pipetting unit 310 can all remain unchanged. Similar to the above-mentioned mixing device 100, the diluting device can also be provided with the initial station 13, the first station 11 and the second station 12. In other embodiments, the initial station 13 can also be omitted.

The mixing assembly 120 is provided on the transport assembly 110. The mixing assembly 120 can carry at least two reactors 20 simultaneously. Take carrying two reactors 20 simultaneously at the same time as an example, one of the reactors 20 is denoted as a first reactor, and the other reactor 20 is referred to as a second reactor. At least two accommodating holes 122a are provided on the mixing assembly 120. The first reactor and the second reactor can be respectively placed in different accommodating holes 122a. The transport assembly 110 drives the mixing assembly 120 to move among the initial station 13, the first station 11 and the second station 12.

During the working process of the diluting device, when the mixing assembly 120 is at the initial station 13, the first reactor is transferred from the supply tray to the mixing assembly 120 through the transferring assembly 220. When the mixing assembly 120 is moved to the first station 11, the sample pipetting unit 411 pipets the sample and adds it into the first reactor. When the mixing assembly 120 is moved to the second station 12, the reagent pipetting unit 310 pipets the diluent and adds it to the first reactor. Then, the sample and the diluent are mixed to form a diluted sample. When the mixing assembly 120 returns to the initial station 13 again, the second reactor is moved into the mixing assembly 120 through the transferring assembly 220. When the mixing assembly 120 is moved to the first station 11 again, the sample pipetting unit 411 transfers a part of the diluted sample in the first reactor to the second reactor. When the mixing assembly 120 is moved to the second station 12 again, the reagent component is pipetted by the reagent pipetting unit 310 and added into the second reactor containing the diluted sample. Then, the diluted sample and the reagent component are mixed. When the mixing assembly 120 is finally moved to the initial station 13, the second reactor is moved into the incubation position 213 of the reaction device 200 through the transferring assembly 220. In other embodiments, the first reactor can be moved to the disposal station and be discarded. According to the above-mentioned operating rules, the diluting device can continuously output the reactor 20 after the diluted sample and the reagent component have been mixed, realizing automatic dilution of the sample.

Further, in order to further improve the automatic dilution efficiency of the sample, at least two mixing assemblies 120 are provided. Each mixing assembly 120 can realize the automatic dilution of the sample. The automatic dilution of the sample can be realized by the mixing assemblies 120 in parallel or in series. Similar to the above-mentioned serial type mixing device, the same transport assembly 110 synchronously drives the mixing assemblies 120 to cyclically reciprocate between the first station 11 and the second station 12. Similar to the above-mentioned parallel type mixing device, at least two transport assemblies 110 are provided. Each transport assembly 110 is provided with the mixing assembly 120 for carrying the reactor 20. Each transport assembly 110 drives the mixing assembly 120 to cyclically reciprocate between the first station 11 and the second station 12.

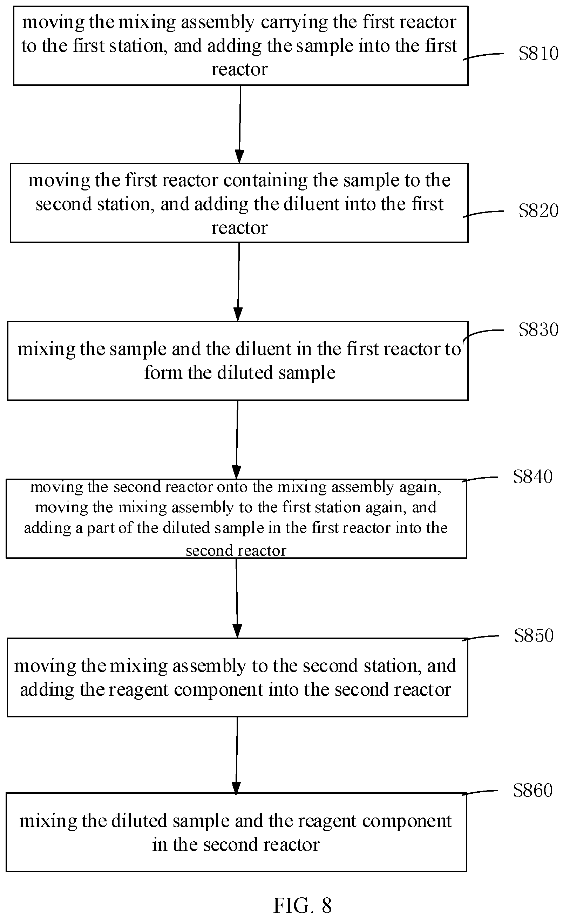

Referring to FIG. 8, when the above-mentioned diluting device is used to realize the automatic dilution of the sample, and to mix the diluted sample and the reagent component, a diluting method can be formed. The diluting method mainly includes the following steps.

At S810, the mixing assembly 120 carrying the first reactor is moved to the first station 11, and then the sample is added into the first reactor 20.

At S820, the first reactor containing the sample is moved to the second station 12, and then the diluent is added into the first reactor.

At S830, the sample and the diluent in the first reactor are mixed to form the diluted sample.

At S840, the second reactor is moved onto the mixing assembly 120 again. Then, the mixing assembly 120 is moved to the first station 11 again, and then a part of the diluted sample in the first reactor 20 is added into the second reactor.

At S850, the mixing assembly 120 is moved to the second station 12, and then the reagent component is added into the second reactor.

At S860, the diluted sample and the reagent component in the second reactor are mixed. After the mixing of the diluted sample and the reagent component is finished, the second reactor is transferred to the incubation position 213 of the reaction device 200.

When at least two mixing assemblies 120 are provided, each mixing assembly 120 can be used in turn in the above-mentioned diluting step. Take providing two mixing assemblies 120 as an example. The first mixing assembly is used when a first sample is automatically diluted, the second mixing assembly is used when a second sample is diluted, the first mixing assembly is used when a third sample is automatically diluted ....

In order to improve working efficiency, both the diluent and the reagent component are placed on the same storage unit 320. After a part of the diluted sample is added into the second reactor, the first reactor is moved out of the mixing assembly 120 and discarded to the disposal station. In other embodiments, in order to achieve solid-liquid separation, the remaining diluted sample in the first reactor can be pipetted first, and then the first reactor in which all the diluted sample is pipetted is discarded.

In order to facilitate the movement of the reactor 20 into or out of the mixing assembly 120, the mixing assembly 120 cyclically reciprocates among the initial station 13, the first station 11 and the second station 12. At the initial station 13, the first and second reactors 20 are moved into or out of the mixing assembly 120. Similarly, the initial station 13, the first station 11 and the second station 12 are arranged on the same straight line, and the initial station 13 is located between the first station 11 and the second station 12. The mixing assembly 120 mixes the sample and the diluent in the first reactor 20, and the diluted sample and the reagent in the second reactor 20 by non-contact eccentric shaking.

It can be seen that, the diluting device of the present application integrates the mixing assemblies 120, and thus it can be moved between different stations to complete the automatic dilution and mixing of the sample, avoiding the pipetting unit to dilute at a fixed station and thus avoiding the reactor from being transferred to another station for mixing, thereby improving the efficiency and effect of dilution and mixing, and solving the high-throughput bottleneck problem of the immune test limited by the automatic dilution of the sample.

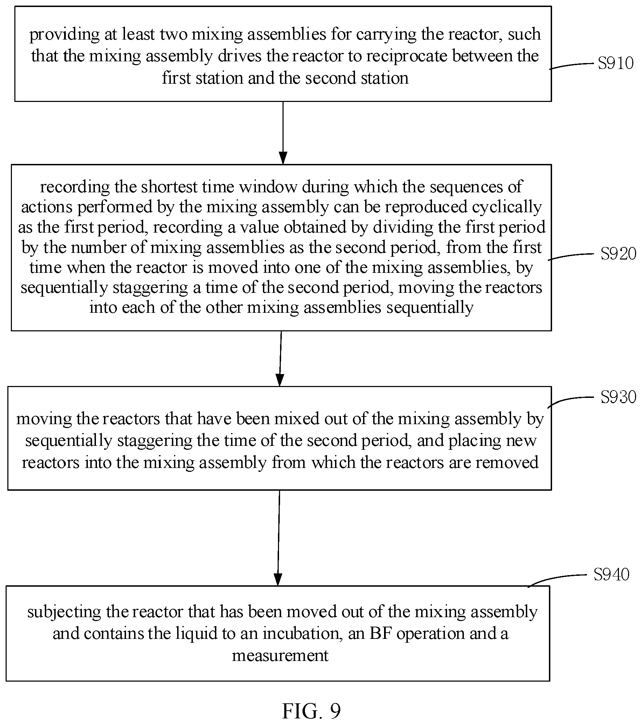

Referring to FIG. 9, an immune analysis method can be formed by using the above-mentioned immunoassay analyzer 10. Take an immune analysis in a one-step reaction mode as an example, the immune analysis method mainly includes the following steps.

At S910, at least two mixing assemblies 120 for carrying the reactor 20 are provided. The mixing assembly 120 drives the reactor 20 to reciprocate between the first station 11 and the second station 12.

At S920, the shortest time window during which the sequences of actions or tasks performed by the mixing assembly 120 can be reproduced cyclically is recorded as the first period. That is, the minimum time interval at which the mixing assembly 120 performs the same action twice in succession is the first period. A value obtained by dividing the first period by the number of mixing assemblies 120 is recorded as the second period. From the first time when the reactor 20 is moved into one of the mixing assemblies 120, the time of the second period is sequentially staggered, such that the reactors 20 are moved into each of the other mixing assemblies 120 sequentially.

At S930, the reactors 20 that have been mixed are moved out of the mixing assembly 120 by sequentially staggering the time of the second period. Then, new reactors 20 are moved into the mixing assembly 120 from which the reactors 20 are removed.

At S940, the reactor 20 that has been moved out of the mixing assembly 120 and contains the reactant is sequentially incubated, subjected to the BF operation, and measured. The incubation time of reactor 20 is 5 to 60 minutes.

It can be understood that the second period is equal to the time between successively outputting two adjacent reactors 20 after the measurement is finished from the reaction device 200, that is, the time between the immunoassay analyzer 10 continuously reporting two adjacent test results.

When performing a test in a reaction mode of other methods, such as the delayed one-step test and the two-step test, in the above step S940, the reactor 20 that has been incubated or washed can be moved into the mixing device 100 again according to steps of S920 and S930. Then, the second reagent is added into the reactor 20, and mixed. After the mixing is finished, the incubation, the BF operation, and the measurement are performed according to step S940.

Specifically, the incubation in step S940 may further include a first incubation and a second incubation as follows.

The first incubation is to incubate the reactor 20 containing the sample and the first reagent for a set time.

The second incubation is to add the second reagent to the reactor 20 subjected to the first incubation and then incubate the reactor 20 for a set time.

When the incubation includes the first incubation and the second incubation, before the step of washing, the reactor 20 subjected to the first incubation is moved into the mixing device 100 again according to steps of S920 and S930. Then, the second reagent is added into the reactor 20, and mixed. After the mixing is finished, the second incubation, the BF operation, and the measurement are performed according to step S940.

The reagent is added into the reactor 20 in two times. The reactor 20 is mixed by the mixing device 100 after each addition of the reagent component. In some embodiments, the immune analysis method further includes the following steps.

The reactor 20 subjected to the first incubation undergoes a first washing.

The reactor 20 subjected to the first washing undergoes the second incubation.

The reactor 20 subjected to the second incubation undergoes a second washing.

Specifically, after the reactor 20 has undergone steps S910, S920, and S930, firstly, the reactor 20 undergoes the first incubation through the reaction device 200, and then the reactor 20 subjected to the first incubation undergoes the first washing through the reaction device 200 for the first time. After the first washing is done, the reactor 20 is moved into the mixing device 100 again according to steps of S920 and S930. Then, the second reagent is added into the reactor 20, and mixed. After the mixing is finished, the incubation, the BF operation, and the measurement are performed according to step S940.

In some embodiments, for example, the same transport assembly 110 drives all the mixing assemblies 120 to move synchronously. That is, the above-mentioned serial type mixing method is used to mix the sample and the reagent in the reactor 20. For another example, multiple transport assemblies 110 are provided. Each transport assembly 110 drives at least one mixing assembly 120 to move. That is, the above-mentioned parallel type mixing method is used to mix the sample and the reagent in the reactor 20.

Referring to the above-mentioned serial type and parallel type mixing methods, the transport assembly 110 can drive the mixing assembly 120 to cyclically reciprocate among the initial station 13, the first station 11 and the second station 12. At the initial station 13, the reactor 20 is moved into or out of the mixing assembly 120. The sample is added into the reactor 20 at the first station 11. The reagent is added into the reactor 20 at the second station 12.

Referring to the structure and working principle of the reaction device 200 described above, the reactor 20 can be moved from the incubation in-out station 15 into the incubation position 213 on the rotating disk 210 for incubation. The reactor 20 is moved from the washing moving-in station 16 into the washing position 212 on the rotating disk 210 for BF operation. Then, the reactor 20 subjected to the BF operation is moved out of the washing position 212 from the washing moving-out station 17. Then, the reactor 20 is moved from the measuring in-out station 18 into the measuring position 211 on the rotating disk 210 for measurement. The moving trajectory of the transferring assembly 220 among the incubation in-out station 15, the washing moving-in station 16, the washing moving-out station 17, and the measuring in-out station 18 is on the same straight line.

The relay station 214 is provided at an inner side of the inner incubation ring of the rotating disk 210 (closest to the rotation center). In particular, the relay station 214 for temporarily carrying the reactor 20 is provided at the rotation center, and two transferring assemblies 220 are provided. The moving trajectory of one of the transferring assemblies 220 on the rotating disk 210 forms a first projection, and the moving trajectory of the other transferring assembly 220 on the rotating disk 210 forms a second projection. The first projection and the second projection are connected into a straight line at the relay station 214. The incubation position 213, the BF operation position, and the measuring position 211 are provided on the same rotating disk 210.

When the measurement is completed, firstly, the waste liquid in the reactor 20 is drawn and removed, and then, the reactor 20 from which the waste liquid is drawn and removed is discarded.

Referring the above reagent pipetting method, when the mixing assembly 120 is at the second station 12, the reagent pipetting unit 310 pipets the reagent from the storage unit 320 and adds it into the reactor 20. Pipetting the reagent includes the following sub-steps.

The reagent pipetting unit 310 and at least two storage units 320 for storing the reagent are provided. The reagent is stored in the reagent containers on the multiple storage portions 321 of the storage unit 320.

The storage portion 321 is moved along with the storage unit 320, such that the reagent pipetting unit 310 pipets the reagent from the reagent container on the storage portion 321 that arrives at the pipetting station 14.

The shortest time window during which the sequences of actions or tasks performed by each storage unit 320 can be reproduced cyclically is equal to the first period. That is, the minimum time interval at which the storage unit 320 performs the same action twice in succession is equal to the first period. From the first time when one of the storage units 320 carrying the reagent is moved towards the pipetting station 14, the time of the second period is sequentially staggered, such that the other storage units 320 carrying the reagent is moved towards the corresponding pipetting station 14.

In some embodiments, when the moving speed of the storage unit 320 does not constitute the bottleneck of the test throughput of the immunoassay analyzer, pipetting the reagent includes the following sub-steps.

The reagent pipetting unit 310 and at least two storage units 320 for storing reagent are provided. The reagent is stored in the reagent containers on the multiple storage portions 321 of the storage unit 320.

The storage portion 321 is moved along with the storage unit 320, such that the reagent pipetting unit 310 pipets the reagent from the reagent container on the storage portion 321 that arrives at the pipetting station 14.

The sequences of actions of the multiple storage units 320 are synchronized in series. That is, the sequences of actions of the multiple storage units 320 in the working period are synchronized, and serialized during the working period. In each working period, each storage unit 320 can position the target storage portion 321 to the pipetting station 14 for the reagent pipetting unit 310 to pipet the reagent. However, only one storage unit 320 is required to position the target storage portion 321 to the pipetting station 14 in each working period for the reagent pipetting unit 310 to pipet the reagent. In short, in any work period, one of the storage units will position the storage portion 321 to the pipetting station 14 for the reagent pipetting unit 310 to pipet the reagent.

The same storage unit 320 contains all the reagent components required by the corresponding analysis item. The number of reagent pipetting units 310 is equal to the number of storage units 320. Each storage unit 320 corresponds to one reagent pipetting unit 310.

Referring to the above diluting method, when the sample is required to be diluted, at the second station 12, before adding other reagent components except for the diluent component into the reactor 20, the diluent is added to the sample in the reactor 20 for dilution, to form the dilution sample.

For a single reactor 20, take the one-step test as an example, a workflow thereof on the immunoassay analyzer 10 is as follows. Firstly, the empty and clean reactor 20 is placed on the mixing assembly 120 located at the initial station 13 from the supply tray through the transferring assembly 220. Secondly, the transport assembly 110 drives the mixing assembly 120 to move to the first station 11, and the sample pipetting unit 411 adds the sample to the reactor 20 located at the first station 11. Thirdly, the transport assembly 110 drives the mixing assembly 120 to move to the second station 12, and the reagent pipetting unit 310 adds the reagent to the reactor 20 located at the second station 12. The mixing assembly 120 mixes the sample and the reagent in the reactor 20. Fourthly, the transferring assembly 220 moves the reactor 20 that has been mixed from the mixing assembly 120 into the incubation position 213 of the rotating disk 210 through the incubation in-out station 15. Fifthly, after the incubation is finished, the transferring assembly 220 moves the reactor 20 out of the incubation position 213 at the incubation in-out station 15 and transfers it from the washing moving-in station 16 to the washing position 212 of the rotating disk 210. Sixthly, after the BF operation is finished, the signal reagent is added into the reactor 20. The transferring assembly 220 moves the reactor 20 out of the washing position 212 at the washing moving-out station 17 and into the signal reagent mixing unit 430 for mixing. Then, the transferring assembly 220 transfers the reactor in which the signal reagent has been mixed from the measuring in-out station 18 to the measuring position 211 of the rotating disk 210. The optical signal in the reactor 20 is measured by the measuring device 230. Seventhly, the waste liquid in the reactor that has been measured is drawn and removed by the waste liquid pipetting assembly 240. Eightly, the transferring assembly 220 moves the reactor 20 out of the rotating disk 210 from the measuring in-out station 18 and discard it to the disposal station.

When performing the delayed one-step test and the two-step test, the transferring assembly 220 can move the reactor 20 that has been incubated or washed into the mixing assembly 120 of the mixing device 100 again. Then, the second reagent is added, and mixed. After the mixing is finished, the transferring assembly 220 moves the reactor 20 that has been mixed to the reaction device 200 for incubation, BF operation, and measurement.

The technical features of the above-mentioned embodiments can be combined arbitrarily. In order to simply the description, all possible combinations of the technical features in the above-mentioned embodiments are not described. However, as long as there is no contradiction in the combinations of these technical features, they should be considered to be fallen into the range described in the present specification.

Only several implementations of the present application are illustrated in the above-mentioned embodiments, and the description thereof is relatively specific and detailed, but it should not be understood as a limitation on the scope of the present application. It should be noted that for those of ordinary skill in the art, without departing from the concept of the present application, several modifications and improvements can be made, which all fall within the protection scope of the present application. Therefore, the protection scope of the present application shall be subject to the appended claims.

## Claims

1. A reaction device (200), comprising:
a rotating disk (210) configured to incubate, wash, and measure a reactant mixed with a sample in a reactor (20);
a washing assembly (250) located above the rotating disk (210), and capable of dispensing a washing solution into the reactor (20) and drawing unbound ingredients in the reactor (20), and the washing assembly (250) comprising a signal reagent dispensing portion configured to dispense a signal reagent into the reactor (20) subjected to a BF operation;
a measuring device (230) connected to the rotating disk (210), and configured to measure an optical signal of the reactant in the reactor (20) after adding the signal reagent; and
a transferring assembly (220) configured to transfer the reactor (20);
wherein the rotating disk (210) comprises an incubation ring (203), a washing ring (202), and a measuring ring (201) that are arranged around a rotation center thereof, the incubation ring (203) is provided with incubation positions (213) that are arranged at intervals along a circumferential direction thereof and configured to incubate the reactant in the reactor (20), the washing ring (202) is provided with washing positions (212) that are arranged at intervals along a circumferential direction thereof and configured to wash the reactant in the reactor (20), the measuring ring (201) is provided with measuring positions (211) that are arranged at intervals along a circumferential direction thereof and configured to measure a signal of the reactant in the reactor (20),
**characterized in that** the device further comprises:
- a signal reagent mixing unit (430) configured to mix the signal reagent in the reactor (20) after adding the signal reagent;
and wherein the transferring assembly (220) is configured to transfer the reactor (20) between the rotating disc (210) and the signal reagent mixing unit (430), and to transfer the reactor (20) between the signal reagent mixing unit (430), the incubation position (213), the washing position (212), and the measuring position (211), wherein
a moving trajectory of the transferring assembly (220) is on the same straight line.

2. The reaction device (200) according to claim 1, wherein the reactor (20) containing the signal reagent is mixed by eccentric shaking.

3. The reaction device (200) according to claim 1, wherein a time for mixing the signal reagent is 2 to 6 seconds.

4. The reaction device (200) according to claim 1, further being provided with an incubation in-out station (15) configured for the reactor (20) to move into and out of the incubation position (213), a washing moving-in station (16) configured for the reactor (20) to move into the washing position (212), a washing moving-out station (17) configured for the reactor (20) to move out of the washing position (212), and a measuring in-out station (18) configured for the reactor (20) to move into and out of the measuring position (211), the transferring assembly (220) moves among the incubation in-out station (15), the washing moving-in station (16), the washing moving-out station (17), and the measuring in-out station (18).

5. The reaction device (200) according to claim 4, wherein the transferring assembly (220) moves the reactor (20) containing the signal reagent out of the washing position (212) from the incubation in-out station (15), and into the signal reagent mixing unit (430).

6. The reaction device (200) according to claim 4, wherein the transferring assembly (220) moves the reactor (20) after the signal reagent has been mixed out of the signal reagent mixing unit (430), and into the measuring position (211) from the measuring in-out station (18).

7. The reaction device (200) according to claim 4, wherein the incubation ring (203), the washing ring (202), and the measuring ring (201) are arranged at intervals from inside to outside around the rotation center.

8. The reaction device (200) according to claim 1, further comprising a waste liquid pipetting assembly (240) located above the measuring ring (201), wherein the waste liquid pipetting assembly (240) is configured to draw and remove the signal reagent in the reactor (20) after the measuring is completed.

9. An immunoassay analyzer (10), comprising the reaction device (200) according to any one of claims 1 to 8.

## Patentansprüche

1. Reaktionsvorrichtung (200), umfassend:
eine Drehscheibe (210), die dafür ausgelegt ist einen Reaktionspartner, welcher in einem Reaktor (20) mit einer Probe gemischt ist, zu inkubieren, zu waschen und zu messen;
eine Waschbaugruppe (250), die sich oberhalb der Drehscheibe (210) befindet und dazu befähigt ist, eine Waschlösung in den Reaktor (20) abzugeben und ungebundene Inhaltsstoffe abzuziehen, die sich in dem Reaktor (20) befinden, wobei die Waschbaugruppe (250) weiterhin einen signalreagenzabgebenden Teilbereich umfasst, der dafür ausgelegt ist, ein Signalreagenz in den Reaktor (20) abzugeben, welcher einem Vorgang des Entfernens ungebundener Inhaltsstoffe (bound-free, BF) unterzogen wurde;
eine Messvorrichtung (230), die mit der Drehscheibe (210) verbunden ist und dafür ausgelegt ist, ein optisches Signal des Reaktionspartners im Reaktor (20) nach dem Zusatz des Signalreagenzes zu messen; und
eine Überführungsbaugruppe (220), die dafür ausgelegt ist, den Reaktor (20) zu überführen;
wobei die Drehscheibe (210) einen Inkubationsring (203), einen Waschring (202) und einen Messring (201) umfasst, die rundum einen Drehmittelpunkt derselben angeordnet sind, der Inkubationsring (203) mit Inkubationspositionen (213) versehen ist, die in Abständen entlang einer Umfangsrichtung desselben angeordnet und dafür ausgelegt sind, den Reaktionspartner in dem Reaktor (20) zu inkubieren, der Waschring (202) mit Waschpositionen (212) versehen ist, die in Abständen entlang einer Umfangsrichtung desselben angeordnet und dafür ausgelegt sind, den Reaktionspartner im Reaktor (20) zu waschen, der Messring (201) mit Messpositionen (211) versehen ist, die in Abständen entlang einer Umfangsrichtung desselben angeordnet und dafür ausgelegt sind, ein Signal des Reaktionspartners im Reaktor (20) zu messen,
**dadurch gekennzeichnet, dass** die Vorrichtung weiterhin Folgendes umfasst:
- und eine Signalreagenz-Mischeinheit (430), die dafür ausgelegt ist, das Signalreagenz in dem Reaktor (20) zu mischen, nachdem das Signalreagenz zugesetzt wurde;
und wobei die Überführungsbaugruppe (220) dafür ausgelegt ist, den Reaktor (20) zwischen der Drehschreibe (210) und der Signalreagenz-Mischeinheit (430) zu überführen und den Reaktor (20) zwischen der Signalreagenz-Mischeinheit (430), der Inkubationsposition (213), der Waschposition (212) und der Messposition (211) zu überführen, wobei
eine Bewegungsstrecke der Überführungsbaugruppe (220) auf derselben geraden Linie liegt.

2. Reaktionsvorrichtung (200) gemäß Anspruch 1, wobei der Reaktor (20), welcher das Signalreagenz enthält, ein Mischen durch exzentrisches Schütteln erfährt.

3. Reaktionsvorrichtung (200) gemäß Anspruch 1, wobei eine Zeit zum Mischen des Signalreagenzes 2 bis 6 Sekunden beträgt.

4. Reaktionsvorrichtung (200) gemäß Anspruch 1, wobei diese weiterhin mit einer Inkubations-Belade/Entladestation (15), die dafür ausgelegt ist, den Reaktor (20) in die Inkubationsposition (213) und aus dieser heraus zu bewegen, einer Wasch-Beladestation (16), die dafür ausgelegt ist, den Reaktor (20) in die Waschposition (212) zu bewegen, einer Wasch-Entladestation (17), die dafür ausgelegt ist, den Reaktor (20) aus der Waschposition (212) heraus zu bewegen, und einer Mess-Belade/Entladestation versehen ist, die dafür ausgelegt ist, den Reaktor (20) in die Messposition (211) und aus dieser heraus zu bewegen, wobei die Überführungsbaugruppe (220) sich zwischen der Inkubations-Belade/Entladestation (15), der Wasch-Beladestation (16), der Wasch-Entladestation (17) und der Mess-Belade/Entladestation (18) bewegt.

5. Reaktionsvorrichtung (200) gemäß Anspruch 4, wobei die Überführungsbaugruppe (220) den Reaktor (20), welcher das Signalreagenz enthält, ausgehend von der Inkubations-Belade/Entladestation (15) aus der Waschposition (212) heraus sowie in die Signalreagenz-Mischeinheit (430) bewegt.

6. Reaktionsvorrichtung (200) gemäß Anspruch 4, wobei die Überführungsbaugruppe (220) den Reaktor (20), nachdem das Signalreagenz gemischt wurde, aus der Signalreagenz-Mischeinheit (430) heraus sowie ausgehend von der Mess-Belade/Entladestation (18) in die Messposition (211) bewegt.

7. Reaktionsvorrichtung (200) gemäß Anspruch 4, wobei der Inkubationsring (203), der Waschring (202) und der Messring (201) in Abständen von innen nach außen rundum den Drehmittelpunkt angeordnet sind.

8. Reaktionsvorrichtung (200) gemäß Anspruch 1, die weiterhin einen Abfallflüssigkeits-Pipettierbaugruppe (240) umfasst, die sich oberhalb des Messrings (201) befindet, wobei die Abfallflüssigkeits-Pipettierbaugruppe (240) dafür ausgelegt ist, das Signalreagenz, welches sich in dem Reaktor (20) befindet, nach Abschluss des Messvorgangs abzuziehen und zu entfernen.

9. Immunoassay-Analysegerät (10), welches die Reaktionsvorrichtung (200) gemäß einem beliebigen der Ansprüche 1 bis 8 umfasst.

## Revendications

1. Dispositif de réaction (200), comprenant :
un disque rotatif (210) configuré pour incuber, nettoyer et mesurer un réactif mélangé à un échantillon dans un réacteur (20) ;
un ensemble de nettoyage (250) situé au-dessus du disque rotatif (210), et capable de distribuer une solution de nettoyage dans le réacteur (20) et d'aspirer des ingrédients non liés dans le réacteur (20), et l'ensemble de nettoyage (250) comprenant une partie de distribution de réactif de signal configurée pour distribuer un réactif de signal dans le réacteur (20) soumis à une opération BF ;
un dispositif de mesure (230) connecté au disque rotatif (210), et configuré pour mesurer un signal optique du réactif dans le réacteur (20) après avoir ajouté le réactif de signal ; et
un ensemble de transfert (220) configuré pour transférer le réacteur (20) ;
dans lequel le disque rotatif (210) comprend un anneau d'incubation (203), un anneau de nettoyage (202) et un anneau de mesure (201) qui sont agencés autour d'un centre de rotation de celui-ci, l'anneau d'incubation (203) est pourvu de positions d'incubation (213) qui sont agencées à des intervalles le long d'une direction circonférentielle de celui-ci et configurées pour incuber le réactif dans le réacteur (20), l'anneau de nettoyage (202) est pourvu de positions de nettoyage (212) qui sont agencées à des intervalles le long d'une direction circonférentielle de celui-ci et configurées pour nettoyer le réactif dans le réacteur (20), l'anneau de mesure (201) est pourvu de positions de mesure (211) qui sont agencées à des intervalles le long d'une direction circonférentielle de celui-ci et configurées pour mesurer un signal du réactif dans le réacteur (20),
**caractérisé en ce que** le dispositif comprend en outre :
- une unité de mélange de réactif de signal (430) configurée pour mélanger le réactif de signal dans le réacteur (20) après avoir ajouté le réactif de signal ;
et dans lequel l'ensemble de transfert (220) est configuré pour transférer le réacteur (20) entre le disque rotatif (210) et l'unité de mélange de réactif de signal (430), et pour transférer le réacteur (20) entre l'unité de mélange de réactif de signal (430), la position d'incubation (213), la position de nettoyage (212) et la position de mesure (211), dans lequel
une trajectoire de déplacement de l'ensemble de transfert (220) est sur la même ligne droite.

2. Dispositif de réaction (200) selon la revendication 1, dans lequel le réacteur (20) contenant le réactif de signal est mélangé par agitation excentrique.

3. Dispositif de réaction (200) selon la revendication 1, dans lequel un temps pour mélanger le réactif de signal est de 2 à 6 secondes.

4. Dispositif de réaction (200) selon la revendication 1, étant en outre pourvu d'une station d'entrée et de sortie d'incubation (15) configurée pour que le réacteur (20) se déplace dans et hors de la position d'incubation (213), d'une station d'entrée de nettoyage (16) configurée pour que le réacteur (20) se déplace dans la position de nettoyage (212), d'une station de sortie de nettoyage (17) configurée pour que le réacteur (20) se déplace hors de la position de nettoyage (212), et une station d'entrée et de sortie de mesure (18) configurée pour que le réacteur (20) se déplace dans et hors de la position de mesure (211), l'ensemble de transfert (220) se déplace entre la station d'entrée et de sortie d'incubation (15), la station d'entrée de nettoyage (16), la station de sortie de nettoyage (17), et la station d'entrée et de sortie de mesure (18).

5. Dispositif de réaction (200) selon la revendication 4, dans lequel l'ensemble de transfert (220) déplace le réacteur (20) contenant le réactif de signal hors de la position de nettoyage (212) à partir de la station d'incubation d'entrée et de sortie (15), et jusque dans l'unité de mélange de réactif de signal (430).

6. Dispositif de réaction (200) selon la revendication 4, dans lequel l'ensemble de transfert (220) déplace le réacteur (20) après que le réactif de signal a été mélangé à partir de l'unité de mélange de réactif de signal (430), et jusque dans la position de mesure (211) à partir de la station d'entrée et de sortie de mesure (18).

7. Dispositif de réaction (200) selon la revendication 4, dans lequel l'anneau d'incubation (203), l'anneau de nettoyage (202) et l'anneau de mesure (201) sont agencés à des intervalles de l'intérieur à l'extérieur autour du centre de rotation.

8. Dispositif de réaction (200) selon la revendication 1, comprenant en outre un ensemble de pipetage de liquide résiduaire (240) situé au-dessus de l'anneau de mesure (201), dans lequel l'ensemble de pipetage de liquide résiduaire (240) est configuré pour aspirer et retirer le réactif de signal dans le réacteur (20) une fois la mesure terminée.

9. Analyseur d'immunoessai (10), comprenant le dispositif de réaction (200) selon l'une quelconque des revendications 1 à 8.
